# EUROPEAN PATENT APPLICATION

(11) **EP 2 481 441 A1**
(43) Date of publication of application: **01.08.2012**
(21) Application number: 12151480.6
(22) Date of filing: 18.01.2012
(51) Int. Cl.: A61M 25/09

(54) **Guidewire**

(30) Priority: 26.01.2011 JP 2011013667
(71) Applicant: Asahi Intecc Co., Ltd., Nagoya-shi, Aichi 463-0024 (JP)
(72) Inventor: Nishigishi, Makoto c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft

(57) **Abstract**

A guidewire includes a core shaft (10) having a first tip portion (11) and a first rear end portion (12), and a coiled body (20) having a second tip portion (21) and a second rear end portion (22) and wound around the outer circumferential surface of the first tip portion (11). The core shaft (10) further includes a first cylindrical portion (13), a second cylindrical portion (14), and a third cylindrical portion (15). The second cylindrical portion (14) has an outer diameter (D₂) larger than that (D₁) of the first cylindrical portion (13) and smaller than the maximum diameter (D_{c}) of the coiled body (20), and the third cylindrical portion (15) has an outer diameter (D₃) larger than that (D₂) of the second cylindrical portion (14) and smaller than the maximum diameter (D_{c}) of the coiled body (20).

## Description

### Cross Reference to Related Applications

This application is based on Japanese Patent Application No. 2011-013667 filed with the Japan Patent Office on January 26, 2011, the entire content of which is hereby incorporated by reference.

### Technical Field

The present invention relates to a guidewire.

### Background Art

In the related art, a guidewire is known as a medical device used for percutaneous coronary intervention (hereinafter simply referred to as "PCI"). The guidewire is used to guide a device such as a balloon and a stent to a lesion site.

Such a guidewire is disclosed in, for example, JP-A-4-309368. This guidewire includes a core shaft and a coiled body. The core shaft has a cylindrical base portion and a tapered tip portion connected to the base portion. The coiled body is wound around the outer circumferential surface of the tip portion. The core shaft and the coiled body are brazed to each other at a predetermined position.
The tip portion of the core shaft corresponds to a distal portion of the guidewire. On the other hand, the base portion of the core shaft corresponds to a proximal portion of the guidewire. The distal portion of the guidewire is inserted into the body. The proximal portion of the guidewire is manipulated by an operator such as a physician.

The distal portion of the guidewire in the related art having such a configuration is flexible so as to minimize damage to an inner wall of a vessel caused by the guidewire inserted into the vessel.

With the guidewire in the related art described in JP-A-4-309368 having the highly flexible tip portion, however, damage to an inner wall of a vessel can be reduced but, on the other hand, it is difficult for the guidewire to penetrate a lesion site.
In particular, it is difficult for this guidewire to penetrate a lesion site of a chronic total occlusion (hereinafter simply referred to as "CTO"). In addition, the guidewire may form a false lumen even if the guidewire enters a CTO lesion site.

### Summary of Invention

Starting out from a guidewire as described in JP-A-4-309368 the present invention has the object to provide a guidewire which is improved as regards penetrability to a CTO lesion site. This object is solved by a guidewire according to claim 1. Advantageous embodiments thereof are subject-matter of dependent claims.

The present inventors have conducted intensive studies to solve the problems of conventional guidewires. They have found, as a result, that a guidewire excellent in penetrability to a CTO lesion site can be obtained by making the diameter of the tip portion larger thereby increasing a torque strength of the tip portion of the core shaft.

Moreover, the present inventors have found that, when a guidewire including a core shaft with a tip portion having a larger diameter is inserted into a guiding catheter and a distal portion of the guidewire (tip portion of the core shaft) is made to reach a CTO lesion site, the stiff tip portion having the larger diameter of the core shaft and the vicinity thereof are likely to be subject to plastic deformation at a greatly bent portion of the guiding catheter near a coronary ostium (hereinafter simply referred to as "bent portion of the guiding catheter"). In other words, the present inventors have found that the core shaft is likely to be subject to plastic deformation at a position away from the distal portion of the guidewire (tip portion of the core shaft).
In addition, the present inventors have also found that the torque strength at the distal portion of the guidewire decreases abruptly as a result of plastic deformation of the core shaft.

The present inventors have conducted further studies on the basis of these findings and, consequently, have completed a guidewire according to the present invention having an excellent torque at a tip portion thereof and being capable of preventing plastic deformation of a core shaft at a position away from a distal portion thereof.

A guidewire according to the present invention includes: a core shaft having a first tip portion and a first rear end portion opposite to the first tip portion; and a coiled body having a second tip portion and a second rear end portion opposite to the second tip portion and wound around an outer circumferential surface of the first tip portion of the core shaft, wherein the core shaft further includes: a first cylindrical portion to which the second rear end portion is fixed; a second cylindrical portion located nearer to the first rear end portion than the first cylindrical portion; and a third cylindrical portion located nearer to the first rear end portion than the second cylindrical portion; wherein the second cylindrical portion has an outer diameter larger than that of the first cylindrical portion and smaller than a maximum diameter of the coiled body; and wherein the third cylindrical portion has an outer diameter larger than that of the second cylindrical portion and smaller than the maximum diameter of the coiled body.

In the guidewire according to the present invention, the ratio of the outer diameters of the first, second, and third cylindrical portions is preferably within a range of 1:1.05:1.1 to 1:2:3.

Preferably, in the guidewire according to the present invention, the outer diameter of the first cylindrical portion is within a range of 0.15 mm to 0.25 mm, the outer diameter of the second cylindrical portion is within a range of 0.20 mm to 0.30 mm, and the outer diameter of the third cylindrical portion is within a range of 0.30 mm to 0.40 mm.

In the guidewire according to the present invention, the ratio of the lengths of the first, second, and third cylindrical portions is preferably within a range of 1:4:45 to 1:7:80.

Preferably, in the guidewire according to the present invention, the length of the first cylindrical portion is within a range of 20 mm to 30 mm, the length of the second cylindrical portion is within a range of 125 mm to 135 mm, and the length of the third cylindrical portion is within a range of 1400 mm to 1600 mm.

In the guidewire according to the present invention, the first tip portion preferably includes: a cylindrical most distal portion; a first tapered portion connected to the most distal portion and having a diameter increasing toward the first rear end portion side; a middle cylindrical portion connected to the first tapered portion; and a second tapered portion connected to the middle cylindrical portion and having a diameter increasing toward the first rear end portion side.

In the guidewire according to the present invention, the length of the middle cylindrical portion is preferably within a range of 2 mm to 5 mm.

### Brief Description of Drawings

The foregoing and other objects, features, aspects and advantages of the invention will become more apparent from the following detailed description when taken in conjunction with the accompanying drawings.
Fig. 1 is a schematic sectional view of a guidewire according to one embodiment of the present invention taken along the longitudinal direction thereof.
Fig. 2 is a partially cutaway enlarged sectional view illustrating a distal portion of the guidewire illustrated in Fig. 1 and the vicinity thereof.

### Description of Embodiments

Preferred embodiments of the present invention will be described below with reference to the accompanying drawings, in which like reference characters designate similar or identical parts throughout the several views thereof.

### (First embodiment)

A guidewire according to a first embodiment of the present invention includes: a core shaft having a first tip portion and a first rear end portion opposite to the first tip portion; and a coiled body having a second tip portion and a second rear end portion opposite to the second tip portion and wound around an outer circumferential surface of the first tip portion of the core shaft, wherein the core shaft further includes: a first cylindrical portion to which the second rear end portion is fixed; a second cylindrical portion located nearer to the first rear end portion than the first cylindrical portion; and a third cylindrical portion located nearer to the first rear end portion than the second cylindrical portion; wherein the second cylindrical portion has an outer diameter larger than that of the first cylindrical portion and smaller than a maximum diameter of the coiled body; and wherein the third cylindrical portion has an outer diameter larger than that of the second cylindrical portion and smaller than the maximum diameter of the coiled body.

This guidewire has an excellent torque strength at the tip portion thereof. Moreover, this guidewire can prevent plastic deformation of the core shaft at a position away from the distal portion thereof.
Typical configurations and effects of this guidewire will be described in detail below with reference to the drawings.

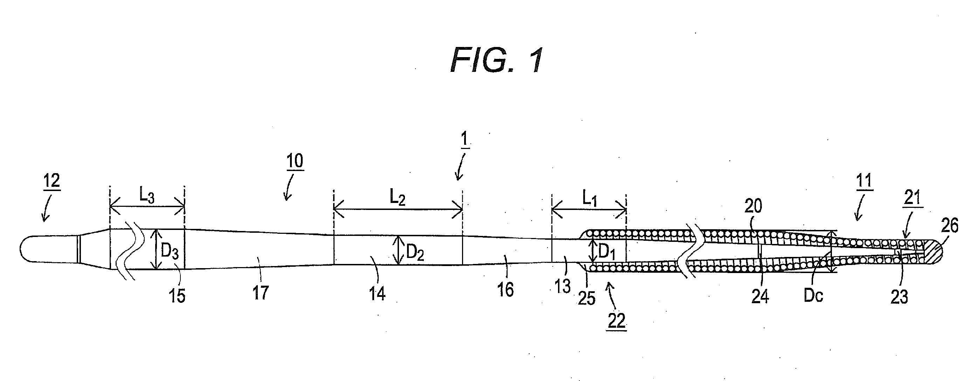

Fig. 1 is a schematic sectional view of a guidewire according to the first embodiment of the present invention taken along the longitudinal direction thereof.

A guidewire 1 according to the first embodiment of the present invention illustrated in Fig. 1 includes a core shaft 10 and a coiled body 20. The core shaft 10 includes a first tip portion 11 and a first rear end portion 12 opposite to the first tip portion 11. The coiled body 20 includes a second tip portion 21 and a second rear end portion 22 opposite to the second tip portion 21. The coiled body 20 is wound around the outer circumferential surface of the first tip portion 11 of the core shaft 10.
To simplify the explanation, a distal portion of the guidewire 1 and the first tip portion of the core shaft 10 are both denoted with the reference numeral 11 in Fig. 1. Similarly, a proximal portion of the guidewire 1 and the first rear end portion of the core shaft 10 are both denoted with the reference numeral 12.

The core shaft 10 includes a first cylindrical portion 13 to which the second rear end portion 22 of the coiled body 20 is fixed, a second cylindrical portion 14 located nearer to the first rear end portion 12 than the first cylindrical portion 13, and a third cylindrical portion 15 located nearer to the first rear end portion 12 than the second cylindrical portion 14.

The outer diameter D₂ of the second cylindrical portion 14 is larger than the outer diameter D₁ of the first cylindrical portion 13 but smaller than the maximum diameter Dc of the coiled body 20.
The outer diameter D₂ of the second cylindrical portion 14 away from the distal portion 11 of the guidewire 1 is larger than the outer diameter D₁ of the first cylindrical portion 13 on the side of the distal portion 11. Accordingly, the guidewire 1 has a higher torque strength than a guidewire having a core shaft with a single diameter having an outer diameter equal to the outer diameter D₁ of the first cylindrical portion 13.
In addition, the outer diameter D₂ of the second cylindrical portion 14 is smaller than the maximum diameter Dc of the coiled body 20. Accordingly, the second cylindrical portion 14 is highly flexible. Thus, the second cylindrical portion 14 and the vicinity thereof are hardly subject to plastic deformation.

The outer diameter D₃ of the third cylindrical portion 15 is larger than the outer diameter D₂ of the second cylindrical portion 14 but smaller than the maximum diameter Dc of the coiled body 20.
The outer diameter D₃ of the third cylindrical portion 15 on the side of the proximal portion 12 of the guidewire 1 is larger than the outer diameter D₂ of the second cylindrical portion 14 on the side of the distal portion 11. Accordingly, the guidewire 1 has a higher torque strength than a guidewire having a core shaft with a single diameter having an outer diameter equal to the outer diameter D₁ of the first cylindrical portion 13 or the outer diameter D₂ of the second cylindrical portion 14.
In addition, the outer diameter D₃ of the third cylindrical portion 15 is smaller than the maximum diameter Dc of the coiled body 20. Accordingly, the third cylindrical portion 15 is highly flexible. Thus, the third cylindrical portion 15 and the vicinity thereof are hardly subject to plastic deformation.

In this manner, the guidewire 1 has an excellent torque transmitting property along its length and can prevent plastic deformation of the core shaft 10.

The guidewire according to the first embodiment of the present invention will be described in more detail below with reference to the drawings.

The first cylindrical portion 13 and the second cylindrical portion 14 are coupled via a first tapered portion 16. The diameter of the first tapered portion 16 decreases toward the first tip portion 11 side. In addition, the second cylindrical portion 14 and the third cylindrical portion 15 are coupled via a second tapered portion 17. The diameter of the second tapered portion 17 decreases toward the first tip portion 11 side.

The outer diameter D₂ of the second cylindrical portion 14 is larger than the outer diameter D₁ of the first cylindrical portion 13 and smaller than the maximum diameter Dc of the coiled body 20. The outer diameter D₃ of the third cylindrical portion 15 is larger than the outer diameter D₂ of the second cylindrical portion 14 but smaller than the maximum diameter Dc of the coiled body 20.

The ratio of the outer diameter D₁ of the first cylindrical portion 13, the outer diameter D₂ of the second cylindrical portion 14 and the outer diameter D₃ of the third cylindrical portion 15 is preferably within a range of 1:1.05:1.1 to 1:2:3 for the following reason.
With the ratio of the outer diameter D₁ of the first cylindrical portion 13, the outer diameter D₂ of the second cylindrical portion 14 and the outer diameter D₃ of the third cylindrical portion 15 being within the above range, a balanced change in the diameter from the first tip portion 11 of the core shaft 10 to the first rear end portion 12 contributes to a higher torque strength applied to the core shaft 10. As a result, it is possible to prevent plastic deformation of the core shaft 10 more reliably.
The outer diameter D₁ of the first cylindrical portion 13 is more preferably within a range of 0.15 to 0.25 mm. The outer diameter D₂ of the second cylindrical portion 14 is more preferably within a range of 0.20 to 0.30 mm. The outer diameter D₃ of the third cylindrical portion 15 is more preferably within a range of 0.30 to 0.40 mm.

The ratio of the length L₁ of the first cylindrical portion 13, the length L₂ of the second cylindrical portion 14 and the length L₃ of the third cylindrical portion 15 is preferably within a rage of 1:4:45 to 1:7:80 for the following reason.
With the ratio of the length L₁ of the first cylindrical portion 13, the length L₂ of the second cylindrical portion 14 and the length L₃ of the third cylindrical portion 15 being within the above range, the second cylindrical portion 14, the second tapered portion 17 or the third cylindrical portion 15, which are less likely to be subject to plastic deformation, is often positioned at the greatly bent portion of the guiding catheter near a coronary ostium when the guidewire 1 according to the present embodiment is inserted into the guiding catheter. It is therefore possible to prevent plastic deformation of the core shaft 10 more reliably.
The length L₁ of the first cylindrical portion 13 is more preferably within a range of 20 to 30 mm. The length L₂ of the second cylindrical portion 14 is more preferably within a range of 125 to 135 mm. The length L₃ of the third cylindrical portion 15 is more preferably within a range of 1400 to 1600 mm.

The interval between an end of the second cylindrical portion 14 on the side of the first tip portion 11 and a most distal portion 26 is preferably within a range of 200 to 300 mm for the following reason.
When the guidewire 1 according to the present embodiment is used for a retrograde approach, the second cylindrical portion 14 or the vicinity thereof is positioned at the bent portion of the guiding catheter. Accordingly, a higher torque is applied to the core shaft 10. As a result, it is possible to prevent plastic deformation of the core shaft 10 more reliably.

Next, a detailed configuration of the distal portion 11 of the guidewire 1 will be described with reference to the drawings.

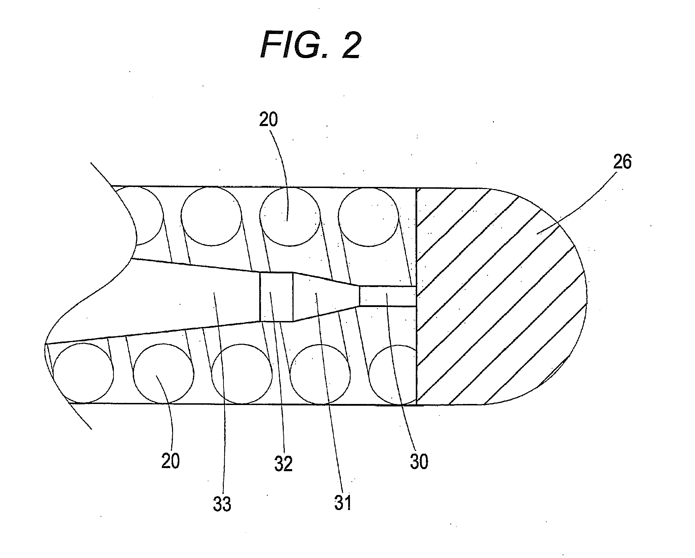

Fig. 2 is a partially cutaway enlarged sectional view illustrating the distal portion 11 of the guidewire 1 according to the present embodiment illustrated in Fig. 1 and the vicinity thereof.

In the distal portion of the guidewire 1 illustrated in Fig. 2, the first tip portion of the core shaft 10 includes a cylindrical most distal portion 30, a first tapered portion 31, a middle cylindrical portion 32 and a second tapered portion 33. The first tapered portion 31 is connected to the most distal portion 30. The diameter of the first tapered portion 31 increases toward the first rear end portion side. The middle cylindrical portion 32 is connected to the first tapered portion 31 and has an outer diameter equal to the maximum diameter of the first tapered portion 31. The second tapered portion 33 is connected to the middle cylindrical portion 32. The diameter of the second tapered portion 33 increases toward the first rear end portion side.
In this manner, the diameter of the first tip portion of the core shaft 10 gradually increases. Therefore, the first tip portion of the core shaft 10 has a higher stiffness and can exhibit a higher torque strength.
More preferably, the middle cylindrical portion 32 has a length (length in the longitudinal direction of the core shaft 10) within a range of 2 to 5 mm.

The most distal portion 30 of the core shaft 10 is bonded to the coiled body 20 at the semispherical most distal portion 26 of the guidewire 1.

Examples of the material for the core shaft 10 illustrated in Fig. 1 include stainless steel; super elastic alloys such as an Ni-Ti alloy; a piano wire; and a tungsten wire.
Examples of the stainless steel include martensite-based stainless steel, ferrite-based stainless steel, austenite-based stainless steel, austenitic-ferritic duplex stainless steel, and precipitation-hardened stainless steel.
Among these, the austenite-based stainless steel is preferable, and more preferable examples thereof include SUS304, SUS316, and SUS316L.
Examples of the material for the most distal portion 26 include super elastic alloys such as an Ni-Ti alloy; a piano wire; a tungsten wire and other materials.

The coiled body 20 includes a strand spirally wound around the outer circumferential surface of the first tip portion 11 of the core shaft 10.
Preferred examples of the material for the strand constituting the coiled body 20 include stainless steel such as manensite-based stainless steel, ferrite-based stainless steel, austenite-based stainless steel, austenitic-ferritic duplex stainless steel, and precipitation-hardened stainless steel; super elastic alloys such as an Ni-Ti alloy; and radiopaque metals such as platinum, gold, and tungsten.
The strand preferably has a diameter within a range of 0.03 to 0.08 mm.

The coiled body 20 preferably has a cross-section, along the longitudinal direction of the guidewire 1, having a tapered shape with the diameter decreasing toward the first tip portion 11.
The coiled body 20 having such a tapered shape facilitates insertion of the guidewire 1 into a CTO lesion site.

The coiled body 20 has a coil tip end brazing portion 23, a coil middle brazing portion 24 located nearer to the second rear end portion 22 than the coil tip end brazing portion 23, and a coil rear end brazing portion 25 located nearer to the second rear end portion 22 than the coil middle brazing portion 24. Theses are brazed to the core shaft 10.
Note that the coil middle brazing portion 24 may be formed at one position or at a plurality of positions.

Examples of the material for brazing metal include aluminum alloy solder, silver solder, gold solder, zinc, Sn-Pb alloy, Pb-Ag alloy, and Sn-Ag alloy.

The outer surface of the guidewire 1 may be coated with a hydrophilic material. This can lower the sliding resistance of the guidewire 1 in the guiding catheter, in a tubular organ, or in an intracorporeal tissue. As a result, the guidewire 1 can be moved smoothly.

Examples of the hydrophilic material include cellulose-based polymeric substance, polyethylene oxide-based polymeric substance, maleic anhydride-based polymeric substance (e.g., maleic anhydride copolymer such as methylvinyl ether-maleic anhydride copolymer), acrylamide-based polymeric substance (e.g., polyacrylamide, and polyglycidyl methacrylate-dimethylacrylamide block copolymer), water-soluble nylon, polyvinyl alcohol, polyvinylpyrrolidone, and hyaluronate.
Among these, hyaluronate is preferable.

The guidewire 1 according to the present embodiment includes the first cylindrical portion 13, the second cylindrical portion 14 and the third cylindrical portion 15 having the predetermined shapes as described above. Accordingly, the distal portion of the guidewire 1 has a higher torque strength and it is possible to prevent plastic deformation of the core shaft 10.

The guidewire 1 of the present embodiment can be produced as follows, for example. First, the core shaft 10 is formed by tapering a rod so that the core shaft 10 has the predetermined shape as described above. Then, the tip portion of the formed core shaft 10 is inserted into the coiled body 20. Then, the core shaft 10 and the coiled body 20 are brazed at (a) predetermined position(s).
Examples of the tapering process include a cutting process such as centering grinding, a swaging process, and a drawing process.

The guidewire 1 of the present embodiment can be suitably used for PCI, for example.
In particular, the guidewire 1 is useful for the retrograde approach technique. According to this technique, the guidewire 1 is inserted into a deeper point in a coronary artery.
This will be explained below.

According to the retrograde approach, the guidewire does not directly approach a stenotic lesion site. The guidewire approaches a lesion site in a direction opposite to that in the conventional technique via a specific vessel (e.g., a thin vessel called a collateral channel).
Specifically, if there is a lesion site in the right coronary artery, the guidewire approaches the lesion site through a right coronary artery ostium according to the antegrade approach that is normally employed. According to the retrograde approach, on the other hand, the guidewire approaches the lesion site through a left coronary artery ostium, passes through the collateral channel and reaches the lesion site.
The retrograde approach is considered to be suitable for treatment of a relatively severe lesion site that is difficult to treat according to the conventional antegrade approach.

In an operation for PCI, the guiding catheter is greatly bent near a coronary ostium. As a result, the tip portion of the guiding catheter is inserted into the coronary ostium. The guidewire is inserted into the guiding catheter.
According to the retrograde approach described above, the guidewire follows a longer detour path. The guidewire is thus inserted to a deeper point (e.g., a site about 300 mm away from the coronary ostium) in the coronary artery. Accordingly, a portion about 300 mm away from the distal portion of the guidewire is greatly bent along the bent portion of the guiding catheter. Thus, with a guidewire of the related art, a portion about 300 mm away from the distal portion is easily subject to plastic deformation.
The guidewire of the present embodiment, however, has the first cylindrical portion, the second cylindrical portion and the third cylindrical portion having the predetermined shapes as described above. It is therefore possible to prevent plastic deformation of the core shaft. Moreover, it is also possible to prevent a great loss in the torque of the distal portion of the guidewire.
As described above, the guidewire according to the present embodiment can be particularly suitably used for the retrograde approach.

### (Another embodiment)

A proximal portion of a guidewire according to the present invention may have an attachment structure for attaching an operating portion such as a torquer. Alternatively, a proximal portion of a guidewire may include an extension wire attaching portion for attaching an extension wire for extending the length of the guidewire.
While the invention has been shown and described in detail, the foregoing description is in all aspects illustrative and not restrictive. It is therefore understood that numerous modifications and variations can be devised without departing from the spirit and scope of the invention.

### Reference Signs List

- 1: guidewire
- 10: core shaft
- 11: first tip portion
- 12: first rear end portion
- 13: first cylindrical portion
- 14: second cylindrical portion
- 15: third cylindrical portion
- 20: coiled body
- 21: second tip portion
- 22: second rear end portion
- 30: most distal portion
- 31: first tapered portion
- 32: middle cylindrical portion
- 33: second tapered portion
- D₁: outer diameter of first cylindrical portion
- D₂: outer diameter of second cylindrical portion
- D₃: outer diameter of third cylindrical portion
- Dc: maximum diameter of coiled body

## Claims

1. A guidewire comprising:
a core shaft (10) having a first tip portion (11) and a first rear end portion (12) opposite to the first tip portion (11); and
a coiled body (20) having a second tip portion (21) and a second rear end portion (22) opposite to the second tip portion (21) and wound around an outer circumferential surface of the first tip portion (11) of the core shaft, **characterized in that**
the core shaft (10) further comprises:
a first cylindrical portion (13) to which the second rear end portion (22) is fixed;
a second cylindrical portion (14) located nearer to the first rear end portion (12) than the first cylindrical portion (13); and
a third cylindrical portion (15) located nearer to the first rear end portion (12) than the second cylindrical portion (14),
wherein the second cylindrical portion (14) has an outer diameter (D₂) larger than that (D₁) of the first cylindrical portion (13) and smaller than a maximum diameter (D_{c}) of the coiled body (20), and
wherein the third cylindrical portion (15) has an outer diameter (D₃) larger than that (D₂) of the second cylindrical portion (14) and smaller than the maximum diameter (D_{c}) of the coiled body (20).

2. The guidewire according to claim 1, wherein a ratio of the outer diameter (D₁) of the first cylindrical portion (13), the outer diameter (D₂) of the second cylindrical portion (14) and the outer diameter (D₃) of the third cylindrical portion (15) is within a range of 1:1.05:1.1 to 1:2:3.

3. The guidewire according to claim 2, wherein the outer diameter (D₁) of the first cylindrical portion (13) is within a range of 0.15 to 0.25 mm, the outer diameter (D₂) of the second cylindrical portion (14) is within a range of 0.20 to 0.30 mm, and the outer diameter (D₃) of the third cylindrical portion (15) is within a range of 0.30 to 0.40 mm.

4. The guidewire according to any one of claims 1 to 3, wherein a ratio of the length (L₁) of the first cylindrical portion (13), the length (L₂) of the second cylindrical portion (14) and the length (L₃) of the third cylindrical portion (15) is within a range of 1:4:45 to 1:7:80.

5. The guidewire according to any one of claims 1 to 4, wherein the length (L₁) of the first cylindrical portion (13) is within a range of 20 to 30 mm, the length (L₂) of the second cylindrical portion (14) is within a range of 125 to 135 mm, and the length (L₃) of the third cylindrical portion (15) is within a range of 1400 to 1600 mm.

6. The guidewire according to any one of claims 1 to 5, wherein the first tip portion (11) comprises:
a cylindrical most distal portion (30);
a first tapered portion (31) connected to the most distal portion (30) and having a diameter increasing toward the first rear end portion (12) side;
a middle cylindrical portion (32) connected to the first tapered portion (31); and
a second tapered portion (33) connected to the middle cylindrical portion (32) and having a diameter increasing toward the first rear end portion (12) side.

7. The guidewire according to claim 6, wherein the middle cylindrical portion (32) has a length within a range of 2 to 5 mm.
